(19) Europäisches Patentamt
European Patent Office
Office européen des brevets

(11) EP 1 487 832 B2

(12) # NEUE EUROPÄISCHE PATENTSCHRIFT

Nach dem Einspruchsverfahren

(45) Veröffentlichungstag und Bekanntmachung des Hinweises auf die Entscheidung über den Einspruch:
**29.08.2018 Patentblatt 2018/35**

(45) Hinweis auf die Patenterteilung:
**08.08.2007 Patentblatt 2007/32**

(21) Anmeldenummer: **03708206.2**

(22) Anmeldetag: **10.03.2003**

(51) Int Cl.:
***C07D 451/10*** ^(2006.01) ***A61P 11/00*** ^(2006.01)
***A61K 31/40*** ^(2006.01)

(86) Internationale Anmeldenummer:
**PCT/EP2003/002422**

(87) Internationale Veröffentlichungsnummer:
**WO 2003/078429 (25.09.2003 Gazette 2003/39)**

(54) **KRISTALLINES MIKRONISAT DES TIOTROPIUMBROMIDS**

MICRONIZED CRYSTALLINE TIOTROPIUM BROMIDE

MICRONISAT CRISTALLIN DE BROMURE DE TIOTROPIUM

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **20.03.2002 DE 10212264**

(43) Veröffentlichungstag der Anmeldung:
**22.12.2004 Patentblatt 2004/52**

(60) Teilanmeldung:
**07102206.5 / 1 785 422**
**16159836.2 / 3 053 921**

(73) Patentinhaber: **Boehringer Ingelheim Pharma GmbH & Co. KG**
**55216 Ingelheim (DE)**

(72) Erfinder:
- **BENDER, Helmut**
  **65193 WIESBADEN (DE)**
- **GRAEBNER, Hagen**
  **55218 INGELHEIM AM RHEIN (DE)**
- **SCHINDLER, Konrad**
  **55218 INGELHEIM AM RHEIN (DE)**
- **TRUNK, Michael, Josef, Friedrich**
  **55218 INGELHEIM AM RHEIN (DE)**
- **WALZ, Michael**
  **55411 BINGEN AM RHEIN (DE)**

(56) Entgegenhaltungen:
**EP-A- 0 418 716** **WO-A2-00/64468**
**CA-A1- 2 368 583** **FR-A- 2 779 347**

Bemerkungen:
Die Akte enthält technische Angaben, die nach dem Eingang der Anmeldung eingereicht wurden und die nicht in dieser Patentschrift enthalten sind.

EP 1 487 832 B2

**Beschreibung**

Die Erfindung betrifft ein Verfahren zur Herstellung eines kristallinen Mikronisats von (1α,2β,4β,5α,7β)-7-[(hydroxydi-2-thienylacetyl)oxy]-9,9-dimethyl-3-oxa-9-azoniatricyclo[3.3.1.0$^{2,4}$]nonane-bromid.

Hintergrund der Erfindung

Die Verbindung (1α,2β,4β,5α,7β)-7-[(hydroxydi-2-thienylacetyl)oxy]-9,9-dimethyl-3-oxa-9-azoniatricyclo [3.3.1.0$^{2,4}$]nonane-bromid, ist aus der Europäischen Patentanmeldung EP 418 716 A1 bekannt und weist die folgende chemische Struktur auf:

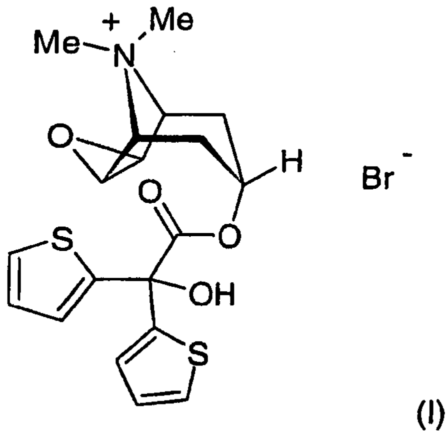

(I)

Die Verbindung besitzt wertvolle pharmakologische Eigenschaften und ist unter dem Namen Tiotropiumbromid (BA679) bekannt. Tiotropiumbromid stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumbromid erfolgt vorzugsweise auf inhalativem Wege. Hierbei können geeignete Inhalationspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten.

Im Hinblick auf die inhalative Applikation von Tiotropiumbromid, ist es erforderlich, den Wirkstoff in feinteiliger (bzw. mikronisierter) Form bereitzustellen. Vorzugsweise weist der Wirkstoff dabei eine mittlere Teilchengröße von 0,5 bis 10μm, bevorzugt von 1 bis 6μm, besonders bevorzugt von 1,5 bis 5μm auf.

Die vorstehend genannten Teilchengrößen werden in der Regel durch Mahlen (sogenanntes Mikronisieren) des Wirkstoffs erzielt. Da als Begleiterscheinung des Mikronisierens trotz der beim Verfahrensablaufs erforderlichen harten Bedingungen ein Abbau des Arzneimittelwirkstoffs weitestgehend vermieden werden muß, stellt eine hohe Stabilität des Wirkstoffs gegenüber dem Mahlvorgang eine unabdingbare Notwendigkeit dar. Dabei muß berücksichtigt werden, daß im Zuge des Mahlvorgangs unter Umständen Veränderungen der Feststoffeigenschaften des Wirkstoffs auftreten können, die einen Einfluß auf die pharmakologischen Eigenschaften der inhalativ zu applizierenden Arzneimittelform haben können. Verfahren zur Mikronisierung von Arzneimittelwirkstoffen sind als solche im Stand der Technik bekannt. Beispielsweise sei diesbezüglich auf FR 2779347 verwiesen, welche ein Verfahren zur Mikronisierung des oral applizierten Wirkstoffs Fenofibrat beschreibt. Es ist nun Aufgabe der vorliegenden Erfindung, ein Verfahren bereitzustellen, welches mikronisiertes Tiotropiumbromid in einer Form zugänglich macht, die den hohen, an einen inhalativ applizierten Wirkstoff zu richtenden Anforderungen genügt und dabei den spezifischen Eigenschaften des Tiotropiumbromids Rechnung trägt.

Detaillierte Beschreibung der Erfindung

Es wurde gefunden, daß Tiotropiumbromid je nach Wahl der Bedingungen, die bei der Reinigung des nach der technischen Herstellung erhaltenen Rohprodukts angewendet werden können, in unterschiedlichen kristallinen Modifikationen, sogenanten Polymorphen, anfällt.

Es wurde ferner gefunden, daß diese unterschiedlichen Modifikationen maßgeblich durch Wahl der zur Kristallisation eingesetzten Lösemittel sowie durch Wahl der beim Kristallisationsprozess gewählten Verfahrensbedingungen gezielt

erhalten werden können.

Für den Zweck der vorliegenden Erfindung, Tiotropiumbromid in für die Inhalation geeigneter, mikronisierter Form bereitzustellen, erwies sich das kristalline Monohydrat des Tiotropiumbromids, welches durch die Wahl spezifischer Reaktionsbedingungen in kristalliner Form erhalten werden kann, besonders geeignet.

Zur Herstellung dieses kristallinen Monohydrats ist es erforderlich, Tiotropiumbromid, welches beispielsweise nach der in der EP 418 716 A1 offenbarten Herstellungsvorschrift erhalten worden ist, in Wasser aufzunehmen, zu Erwärmen, eine Reinigung mit Aktivkohle durchzuführen und nach Abtrennen der Aktivkohle unter langsamem Abkühlen das Tiotropiumbromid-monohydrat langsam zu kristallisieren. Erfindungsgemäß bevorzugt wird wie nachfolgend beschrieben vorgegangen. In einem geeignet dimensionierten Reaktionsgefäß wird das Lösemittel mit Tiotropiumbromid, welches beispielsweise nach der in der EP 418 716 A1 offenbarten Herstellungsvorschrift erhalten worden ist, gemischt. Pro Mol eingesetztes Tiotropiumbromid werden 0,4 bis 1,5 kg, bevorzugt 0,6 bis 1 kg, besonders bevorzugt ca. 0,8 kg Wasser als Lösemittel verwendet.

Die erhaltene Mischung wird unter Rühren erwärmt, vorzugsweise auf mehr als 50°C, besonders bevorzugt auf mehr als 60°C. Die maximal wählbare Temperatur bestimmt sich durch den Siedepunkt des verwendeten Lösemittels Wasser. Vorzugsweise wird die Mischung auf einen Bereich von 80-90°C erhitzt.

In diese Lösung wird Aktivkohle, trocken oder wasserfeucht, eingebracht. Bevorzugt werden pro Mol eingesetztes Tiotropiumbromid 10 bis 50 g, besonders bevorzugt 15 bis 35 g, höchst bevorzugt etwa 25 g Aktivkohle eingesetzt. Gegebenenfalls wird die Aktivkohle vor Einbringen in die Tiotropiumbromid-haltige Lösung in Wasser aufgeschlämmt. Pro Mol eingesetztes Tiotropiumbromid werden zum Aufschlämmen der Aktivkohle 70 bis 200 g, bevorzugt 100 bis 160 g, besonders bevorzugt ca. 135 g Wasser verwendet. Wird die Aktivkohle vor Einbringen in die Tiotropiumbromid-haltige Lösung zuvor in Wasser aufgeschlämmt, empfiehlt es sich, mit der gleichen Menge Wasser nachzuspülen.

Bei konstanter Temperatur wird nach erfolgter Aktivkohlezugabe zwischen 5 bis 60 Minuten, bevorzugt zwischen 10 und 30 Minuten, besonders bevorzugt etwa 15 Minuten weitergerührt und die erhaltene Mischung filtriert, um die Aktivkohle zu entfernen. Der Filter wird anschließend mit Wasser nachgespült. Hierfür werden pro Mol eingesetztes Tiotropiumbromid 140 bis 400 g, bevorzugt 200 bis 320 g, höchst bevorzugt ca. 270 g Wasser verwendet.

Das Filtrat wird anschließend langsam abgekühlt, vorzugsweise auf eine Temperatur von 20-25°C. Die Abkühlung wird vorzugsweise mit einer Abklühlrate von 1 bis 10°C pro 10 bis 30 Minuten, bevorzugt von 2 bis 8°C pro 10 bis 30 Minuten, besonders bevorzugt von 3 bis 5°C pro 10 bis 20 Minuten, höchst bevorzugt von 3 bis 5°C pro ca. 20 Minuten durchgeführt. Gegebenenfalls kann sich nach dem Abkühlen auf 20 bis 25°C eine weitere Abkühlung auf unter 20°C, besonders bevorzugt auf 10 bis 15°C anschließen.

Nach erfolgter Abkühlung wird zwischen 20 Minuten und 3 Stunden, vorzugsweise zwischen 40 Minuten und 2 Stunden, besonders bevorzugt etwa eine Stunde zur Vervollständigung der Kristallisation nachgerührt.

Die entstandenen Kristalle werden abschließend durch Filtrieren oder Absaugen des Lösemittels isoliert. Sollte es erforderlich sein, die erhaltenen Kristalle einem weiteren Waschschritt zu unterwerfen, empfiehlt es sich als Waschlösemittel Wasser oder Aceton zu verwenden. Pro Mol eingesetztes Tiotropiumbromid können zum Waschen der erhaltenen Tiotropiumbromid-monohydrat-Kristalle 0,1 bis 1,0 L, bevorzugt 0,2 bis 0,5 L, besonders bevorzugt etwa 0,3 L Lösemittel Verwendung finden. Gegebenenfalls kann der Waschritt wiederholt durchgeführt werden.

Das erhaltene Produkt wird im Vakuum oder mittels erwärmter Umluft bis zum Erreichen eines Wassergehalts von 2,5 - 4,0 % getrocknet.

Das so erhaltene kristalline Tiotropiumbromid-Monohydrat wird in den nachfolgend beschriebenen Mahlprozess (Mikronisierung) eingesetzt. Zur Durchführung dieses Prozesses können gängige Mühlen zum Einsatz gelangen. Bevorzugt wird die Mikronisierung dabei unter Feuchtigkeitsausschluß durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinenderprallen der Partikel miteinender sowie Aufprall der Partikel auf die Wände des Mahlbehälters erfolgt. Als Mahlgas gelangt erfindungsgemäß bevorzugt Stickstoff zur Anwendung. Das Mahlgut wird mittels des Mahlgases unter spezifischen Drücken (Mahldruck) gefördert. Im Rahmen der vorliegenden Erfindung wird der Mahldruck üblicherweise auf einen Wert zwischen 2 und 8 bar, bevorzugt zwischen 3 und 7 bar, besonders bevorzugt zwischen 3,5 und 6,5 bar eingestellt. Der Eintrag des Mahlgutes in die Luftstrahlmühle erfolgt mittels des Speisegases unter spezifischen Drücken (Speisedruck). Im Rahmen der vorliegenden Erfindung hat sich eine Speisedruck zwischen 2 und 8 bar, bevorzugt zwischen 3 und 7 bar, besonders bevorzugt zwischen 3,5 und 6 bar bewährt. Als Speisegas gelangt vorzugsweise ebenfalls ein Inertgas, besonders bevorzugt ebenfalls Stickstoff zur Anwendung. Die Zufuhr des Mahlguts (kristallines Tiotropiumbromidmonohydrat) kann dabei in einer Förderräte von 5-35 g/min, vorzugsweise mit 10-30 g/min erfolgen.

Beispielsweise und ohne den Gegenstand der Erfindung darauf zu beschränken, hat sich als eine mögliche Ausführungsform einer Luftstrahlmühle das folgende Gerät bewährt: 2-Zoll Microniser mit Mahlring 0,8 mm-Bohrung, Firma Sturtevant Inc., 348 Circuit Street, Hanover, MA 02239, USA. Unter Verwendung dieses Geräts wird der Mahlprozess vorzugsweise mit folgenden Mahlparametern durchgeführt: Mahldruck: 4,5 - 6,5 bar; Speisedruck: 4,5 - 6,5 bar: Zufuhr des Mahlguts: 17 - 21 g/min.

Das so erhaltene Mahlgut wird anschließend unter den nachfolgend genannten spezifischen Bedingungen weiterverarbeitet. Hierzu wird das Mikronisat bei einer Temperatur von 15-40 °C, vorzugsweise 20 - 35 °C, besonders bevorzugt bei 25 - 30 °C Wasserdampf einer relativen Feuchte von wenigstens 40% ausgesetzt. Vorzugsweise wird die Feuchte auf einen Wert von 50 - 95% r.F., bevorzugt auf 60 - 90 % r.F., besonders bevorzugt auf 70 - 80 % r.F. eingestellt. Unter relativer Feuchte (r.F.) wird im Rahmen der vorliegenden Erfindung der Quotient des Partialdruckes des Wasserdampfs und des Dampfdruckes des Wassers bei der betreffenden Temperatur verstanden. Vorzugsweise wird das aus vorstehend beschriebenem Mahlprozess erhältliche Mikronisat den oben genannten Raumbedingungen wenigstens über einen Zeitraum von 6 Stunden ausgesetzt. Bevorzugt wird das Mirkonisat den genannten Raumbedingungen allerdings für 12 bis 48 Stunden, vorzugsweise 18 bis 36 Stunden, besonders bevorzugt 20 bis 28 Stunden ausgesetzt.

Das gemäß vorstehender Vorgehensweise erhältliche Mikronisat des Tiotropiumbromids weist eine charakteristische Partikelgröße $X_{50}$ von zwischen 1,0 μm und 3,5 μm, bevorzugt zwischen 1,1μm und 3,3 μm, besonders bevorzugt zwischen 1,2 μm und 3,0μm und $Q_{(5.8)}$ von größer 60%, bevorzugt größer 70 %, besonders bevorzugt größer 80% auf. Dabei bezeichnet der Kennwert $X_{50}$ den Medianwert der Teilchengröße, unterhalb derer 50% der Teilchenmenge bezüglich der Volumenverteilung der einzelnen Teilchen liegt. Der Kennwert $Q_{(5.8)}$ entspricht der Teilchenmenge der Partikel, die bezogen auf die Volumenverteilung der Partikel unterhalb von 5.8 μm liegt. Die Partikelgrößen wurden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Ebenso charakteristisch für das Tiotropium-Mikronisat, das nach obigem Prozeß dargestellt wurde, sind Spezifische Oberflächenwerte im Bereich zwischen 2 $m^2$/g und 5 $m^2$/g, im besonderen Maße Werte zwischen 2,5 $m^2$/g und 4,5 $m^2$/g und in besonders herausragendem Maße zwischen 3,0 $m^2$/g und 4,0 $m^2$/g.

Die Durchführung des erfindungsgemäßen Prozesses führt zu einem Mikronisat des Tiotropiumbromids, welches durch spezifische Lösungswärmen gekennzeichnet ist. Diese weisen vorzugsweise einen Wert von größer 65 Ws/g, bevorzugt größer 71 Ws/g auf. Besonders bevorzugt übersteigt der Wert der Lösungswärme des erfindungsgemäßen Mikronisats den Betrag von 74 Ws/g.

Detaillierte Angaben zur Bestimmung der Lösungsenthalpien sind den experimentellen Beschreibungen der Erfindung zu entnehmen.

Das Tiotropiumbromid-mikronisat, das mit Hilfe des obigen Verfahrens gewonnen werden kann, zeichnet sich ferner dadurch aus, daß der Wassergehalt des Mikronisats zwischen 1 % und 4,5 %, bevorzugt zwischen 1,4% und 4,2% besonders bevorzugt zwischen 2,4% und 4,1% liegt. Besonders bevorzugtes Tiotropiumbromid-mikronisat ist dadurch gekennzeichnet, daß der Wassergehalt des Mikronisats zwischen 2,6 % und 4,0 %, besonders bevorzugt zwischen 2,8% und 3,9% besonders bevorzugt zwischen 2,9% und 3,8% liegt.

Im Rahmen der vorliegenden Erfindung ist, soweit nichts anderes angegeben, eine Bezugnahme auf Tiotropiumbromid-mikronisat als Bezugnahme auf jenes kristalline Mikronisat des Tiotropiumbromids zu verstehen, welches die vorstehend genannten Charakteristika aufweist und welches gemäß dem zuvor beschriebenen erfindungsgemäßen Verfahren (Mikronisierung und anschließende Weiterbehandlung gemäß den zuvor beschriebenen Parametern) erhältlich ist.

Aufgrund der anticholinergen Wirksamkeit des gemäß vorstehender Vorgehensweise erhältlichen Tiotropiumbromid-mikronisats kann dies zur Herstellung eines Arzneimittels zur Behandlung von Erkrankungen verwendet werden, in denen die Applikation eines Anticholinergikums einen therapeutischen Nutzen entfalten kann. Bevorzugt ist die entsprechende Verwendung zur Herstellung eines Arzneimittels zur Behandlung von Asthma oder COPD.

Das gemäß dem erfindungsgemäßen Verfahren zugängliche Tiotropiumbromid-Mikronisat ist in herausragender Weise zur Darstellung pharmazeutischer Formulierungen geeignet. Besonders bevorzugt kann es zur Herstellung von Inhalationspulvern Verwendung finden, wie z.B. Inhalationspulver enthaltend wenigstens etwa 0,03 %, vorzugsweise unter 5 %, besonders bevorzugt unter 3 % des nach vorstehend beschriebenem Verfahren erhältlichen Tiotropiumbromid-mikronisats im Gemisch mit einem physiologisch unbedenklichen Hilfsstoff, dadurch gekennzeichnet, daß der Hilfsstoff aus einem Gemisch von gröberem Hilfsstoff mit einer mittleren Teilchengröße von 15 bis 80μm und feinerem Hilfststoff mit einer mittleren Teilchengröße von 1 bis 9 μm besteht, wobei der Anteil von feinerem Hilfsstoff an der Gesamthilfsstoffmenge 1 bis 20% beträgt.

Bei den vorstehend genannten prozentualen Angaben, handelt es sich um Gewichtsprozent.

Bevorzugt sind Inhalationspulver, die etwa 0,05 bis etwa 1%, bevorzugt etwa 0,1 bis etwa 0,8%, besonders bevorzugt etwa 0,2 bis etwa 0,5% Tiotropiumbromid-mikronisat enthalten, welches nach vorstehend beschriebenem Verfahren erhältlich ist und die kennzeichnenden Charkteristika des erfindungsgemäß erhältlichen Mikronisats aufweist.

Die das nach dem vorstehend beschriebenen Verfahren erhältliche Tiotropiumbromid-mikronisat enthaltenden Inhalationspulver sind bevorzugt dadurch gekennzeichnet, daß der Hilfsstoff aus einem Gemisch von gröberem Hilfsstoff mit einer mittleren Teilchengröße von 17 bis 50μm, besonders bevorzugt von 20 bis 30μm und feinerem Hilfsstoff mit einer mittleren Teilchengröße von 2 bis 8μm, besonders bevorzugt von 3 bis 7μm besteht. Dabei wird unter der mittleren Teilchengröße im hier verwendeten Sinne der 50%-Wert aus der Volumenverteilung gemessen mittels Laserbeugung nach der Trockendispersionsmethode verstanden. Bevorzugt sind Inhalationspulver bei denen der Anteil von feinerem

Hilfsstoff an der Gesamthilfsstoffmenge 3 bis 15%, besonders bevorzugt 5 bis 10% beträgt.

Wird im Rahmen der vorliegenden Erfindung auf die Bezeichnung Gemisch Bezug genommen, so ist hierbei stets eine Mischung zu verstehen, die durch Mischen zuvor klar definierter Komponenten erhalten wurde. Entsprechend sind beispielsweise als Hilfsstoffgemisch aus gröberen und feineren Hilfsstoffanteilen nur solche Gemische zu verstehen, die durch Mischen einer gröberen Hilfsstoffkomponente mit einer feineren Hilfsstoffkomponente erhalten werden.

Die gröberen und feineren Hilfsstoffanteile können aus dem chemisch gleichen oder aus chemisch verschiedenen Substanzen bestehen, wobei Inhalationspulver, bei denen der gröbere Hilfsstoffanteil und der feinere Hilfsstoffanteil aus der selben chemischen Verbindung bestehen bevorzugt sind.

Als physiologisch unbedenkliche Hilfsstoffe, die zur Darstellung der das nach dem vorstehend beschriebenen Verfahren erhältliche Tiotropiumbromid-mikronisat enthaltendenInhalationspulver zur Anwendung gelangen können seien beispielsweise genannt: Monosaccharide (z.B. Glucoseoder Arabinose), Disaccharide (z.B. Lactose, Saccharose, Maltose oder Trehalose), Oligo- und Polysaccharide (z.B.Dextrane), Polyalkohole (z.B. Sorbit, Mannit, Xylit), Salze (z.B. Natriumchlorid, Calciulcarbonat) oder Mischungen dieserHilfsstoffe miteinander. Bevorzugt gelangen Mono- oder Disaccharide zur Anwendung, wobei die Verwendung vonLactose, Glu - cose oder Trehalose, bevorzugt Lactose oder Glucose, insbesondere, aber nicht ausschließlich in Formihrer Hydrate, bevorzugt ist. Als besonders bevorzugt im Sinne der Erfindung gelangt Lactose, höchst bevorzugt Lactose-monohydrat als Hilfsstoff zur Anwendung.

Die das nach dem vorstehend beschriebenen Verfahren erhältliche Tiotropiumbromid-mikronisat enthaltenden Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren. Vorzugsweise werden die Inhalationspulver allerdings in Kapseln abgefüllt (zu sogenannten Inhaletten), die in Inhalatoren wie beispielsweise in der WO 94/28958 beschrieben, zur Anwendung gelangen. Sollen die erfindungsgemäßen Inhalationspulver im Sinne der vorstehend gennannten bevorzugten Anwendung in Kapseln (Inhaletten) sowie sonstigen Verpackungsformen, die Einzeldosen anbieten, abgefüllt werden, bieten sich Füllmengen von 1 bis 15mg, bevorzugt 3 bis 10mg, höchst bevorzugt von 4 bis 6mg Inhalationspulver pro Kapsel an.

Die das nach dem vorstehend beschriebenen Verfahren erhältliche Tiotropiumbromid-mikronisat enthaltenden Inhalationspulver sind durch ein hohes Maß an Homogenität im Sinne der Einzeldosierungsgenauigkeit gekennzeichnet. Diese liegt in einem Bereich von <8%, bevorzugt < 6% , besonders bevorzugt < 4%.

Die das nach dem vorstehend beschriebenen Verfahren erhältliche Tiotropiumbromid-mikronisat enthaltenden Inhalationspulver sind gemäß der nachfolgend beschriebenen Vorgehensweise erhältlich.

Nach Einwaage der Ausgangsmaterialien erfolgt zunächst die Fertigung der Hilfsstoffmischung aus den definierten Fraktionen des gröberen Hilfsstoffs und des feineren Hilfsstoffs. Anschließend erfolgt die Herstellung der Inhalationspulver aus der Hilfsstoffmischung und dem Wirkstoff. Soll das Inhalationspulver mittels Inhaletten in hierzu geeigneten Inhalatoren appliziert werden, schließt sich der Herstellung der Inhalationspulver die Fertigung der pulverhaltigen Kapseln an.

Bei den nachstehend beschriebenen Herstellverfahren werden die genannten Komponenten in den Gewichtsanteilen eingesetzt, wie sie in den zuvor beschriebenen Zusamensetzungen der erfindungsgemäßen Inhalationspulver beschrieben wurden.

Die Herstellung der Inhaltionspulver erfolgt durch Mischen der gröberen Hilfsstoffanteile mit den feineren Hilfststoffanteilen und andschließendem Mischen der so erhaltenen Hilfsstoffgemische mit dem Wirkstoff.

Zur Herstellung der Hilfsstoffmischung werden die gröberen und feineren Hilfsstoffanteile in einen geeigneten Mischbehälter eingebracht. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der gröbere Hilfsstoff vorgelegt und anschließend der feinere Hilfsstoffanteil in den Mischbehälter eingebracht. Bevorzugt erfolgt bei diesem Mischverfahren die Zugabe der beiden Komponenten portionsweise, wobei ein Teil des gröberen Hilfsstoffs zunächst vorgelegt und anschließend abwechselnd feinerer und gröberer Hilfsstoff zugegeben wird. Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 15 bis 45, besonders bevorzugt in je 20 bis 40 Schichten. Der Mischvorgang der beiden Hilfsstoffe kann bereits während der Zugabe der beiden Komponenten erfolgen. Vorzugsweise wird allerdings erst nach dem schichtweisen Einsieben der beiden Bestandteile gemischt.

Nach Herstellung der Hilfsstoffmischung werden diese und der Wirkstoff, das nach dem vorstehend beschriebenen Verfahren erhältliche Tiotropiumbromid-mikronisat, in einen geeigneten Mischbehälter eingebracht. Der verwendete Wirkstoff weist eine mittlere Teilchengrößevon 0,5 bis 10μm, vorzugsweise von 1 bis 6μm, besonders bevorzugt von 1,5 bis 5μm auf. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird die Hilfsstoffmischung vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist bei der Herstellung der Hilfsstoffmischung das abwechselnde, schichtweise Einsieben der beiden Komponenten. Vorzugsweise

erfolgt das Einsieben der beiden Komponenten abwechselnd in je 25 bis 65, besonders bevorzugt in je 30 bis 60 Schichten. Der Mischvorgang der Hilfsstoffmischung mit dem Wirkstoff kann bereits während der Zugabe der beiden Komponenten erfolgen. Vorzugsweise wird allerdings erst nach dem schichtweisen Einsieben der beiden Bestandteile gemischt.
Die so erhaltene Pulvermischung kann gegebenenfalls erneut ein- oder mehrfach über einen Siebgranulator gegeben und jeweils anschließend einem weiteren Mischvorgang unterworfen werden.
Die folgenden, detaillierten experimentellen Ausführungen dienen einer weitergehenden Erläuterung der vorliegenden Erfindung, ohne den Umfang der Erfindung allerdings auf die nachfolgenden beispielhaften Ausführungsformen zu beschränken.

## Experimenteller Teil

### A) Darstellung von kristallinem Tiotropiumbromid-monohydrat

In einem geeigneten Reaktionsgefäß werden in 25,7 kg Wasser 15,0 kg Tiotropiumbromid eingetragen, welches beispielsweise gemäß der in der Europäischen Patentanmeldung EP 418 716 A1 offenbarten experimentellen Vorgehensweise erhältlich ist. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet. Ausbeute : 13,4 kg Tiotropiumbromid-monohydrat (86 % d. Th.)

### B) Charakterisierung des kristallinen Tiotropiumbromid-monohydrats

Das gemäß vorstehend beschriebener Vorgehensweise erhältliche Tiotropiumbromid-monohydrat wurde einer Untersuchung mittels DSC (Differential Scanning Calorimetry) unterworfen. Das DSC- diagramm weist zwei charakteristische Signale auf. Das erste, relativ breite, endotherme Signal zwischen 50-120°C ist auf die Entwässerung des Tiotropiumbomid-monohydrats zur wasserfreien Form zurückzuführen. Das zweite, relativ scharfe, endotherme Maximum bei 230 ± 5°C ist dem Schmelzen der Substanz zuzuordnen. Diese Daten wurden mittels eines Mettler DSC 821 erhalten und mit dem Mettler Software-Paket STAR ausgewertet. Die Daten wurden bei einer Heizrate von 10 K/min erhoben.
Da die Substanz unter Zersetzung schmilzt (= inkongruenter Schmelzvorgang), hängt der beobachtete Schmelzpunkt sehr von der Heizrate ab. Bei geringeren Heizraten wird der Schmelz-/Zersetzungsvorgang bei deutlich niedrigeren Temperaturen beobachtet, so zum Beispiel mit einer Heizrate von 3 K/min bei 220 ± 5 °C. Es kann außerdem vorkommen, daß der Schmelzpeak aufgespalten vorliegt. Die Aufspaltung tritt umso stärker auf, je geringer die Heizrate im DSC-Experiment ist.
Das kristalline Tiotropiumbromid-monohydrat wurde mittels IR-Spektroskopie charakterisiert. Die Daten wurden mittels eines Nicolet FTIR Spektrometers erhoben und mit dem Nicolet Softwarepaket OMNIC, version 3.1 ausgewertet. Die Messung wurde mit 2,5$\mu$mol Tiotropiumbromid-monohydrat in 300 mg KBr durchgeführt.
Tabelle 1 faßt einige der wesentlichen Banden des IR-Spektrums zusammen.

Tabelle 1: Zuordnung von spezifischen Banden

| Wellenzahl ($cm^{-1}$) | Zuordnung | Schwingungstyp |
|---|---|---|
| 3570, 3410 | O-H | Streckschwingung |
| 3105 | Aryl C-H | Streckschwingung |
| 1730 | C=O | Streckschwingung |
| 1260 | Epoxid C-O | Streckschwingung |
| 1035 | Ester C-OC | Streckschwingung |
| 720 | Thiophen | Ringschwingung |

Das kristalline Tiotropiumbromid-monohydrat wurde mittels Röntgenstrukturanalyse charakterisiert. Die Röntgenbeugungsintensitätsmessungen wurden auf einem AFC7R- 4-Kreisdiffraktometer (Rigaku) unter Verwendung von monochromatisierter Kupfer Kα-Strahlung durchgeführt. Die Strukturlösung und Verfeinerung der Kristallstruktur erfolgte mittels Direkter Methoden (Programm SHELXS86) und FMLQ-Vefeinerung (Programm TeXsan). Experimentelle Details zur Kristallstruktur, Strukturlösung und -Verfeinerung sind in Tabelle 2 zusammengefaßt.

Tabelle 2: Experimentelle Daten zur Kristallstrukturanalyse von Tiotropiumbromid-monohydrat.

A. Kristalldaten

| | |
|---|---|
| Empirische Formel | $[C_{19}H_{22}NO_4S_2]\,Br \cdot H_2O$ |
| Formelgewicht | 472.43 + 18.00 |
| Kristallfarbe, -gestalt | farblos, prismatisch |
| Kristallabmessungen | 0.2 x 0.3 x 0.3 mm |
| Kristallsystem | monoklin |
| Gittertyp | primitv |
| Raumgruppe | $P\ 2_1/n$ |
| Gitterkonstanten | a = 18.0774 Å,<br>b = 11.9711 Å<br>c = 9.9321 Å<br>$\beta$=102.691°<br>V = 2096.96 $Å^3$ |
| Formeleinheiten pro Elementarzelle | 4 |

B. Intensitätsmessungen

| | |
|---|---|
| Diffraktometer | Rigaku AFC7R |
| Röntgengenerator | Rigaku RU200 |
| Wellenlänge | $\lambda$ = 1.54178Å (monochromatisierte Kupfer Kα-Strahlung) |
| Strom, Spannung | 50kV, 100mA |
| Take-off Winkel | 6° |
| Kristallmontage | wasserdampfgesättigte Kapillare |
| Kristall-Detektor-Abstand | 235mm |
| Detektor Öffnung | 3.0 mm vertikal und horizontal |
| Temperatur | 18° |
| Bestimmung der Gitterkonstanten | 25 Reflexe (50.8° < $2\Theta$ < 56.2°) |
| Scan Typ | $\omega$-$2\Theta$ |
| Scan Geschwindigkeit | 8.0 32.0°/min in $\omega$ |
| Scan Breite | (0.58 + 0.30 tan $\Theta$)° |
| $2\Theta$max | 120° |
| Messungen | 5193 |
| Unabhängige Reflexe | 3281 ( Rint=0.051) |
| Korrrekturen | Lorentz-Polarisation<br>Absorption (Transmissionsfaktoren 0.56-1.00) Kristall-decay 10.47% Abfall |

C. Verfeinerung

| | |
|---|---|
| Reflexe (I > $3\sigma$I) | 1978 |

(fortgesetzt)

| | |
|---|---|
| Variable | 254 |
| Verhältnis Reflexe/Parameter | 7.8 |
| R-Werte: R, Rw | 0.062, 0.066 |

Die durchgeführte Röntgenstrukturanlyse ergab, daß kristallines Toptropiumbromidhydrat eine einfache monoklinische Zelle mit folgenden Dimensionen aufweist: a = 18.0774 Å, b = 11.9711 Å, c = 9.9321 Å, β = 102.691 °, V = 2096.96 Å$^3$.

Durch die vorstehende Röntgenstrukturanalyse wurden die in Tabelle 3 beschriebenen Atomkoordinaten bestimmt:

Tabelle 3: Koordinaten

| Atom | x | y | z | u (eq) |
|---|---|---|---|---|
| Br(1) | 0.63938(7) | 0.0490(1) | 0.2651(1) | 0.0696(4) |
| S(1) | 0.2807(2) | 0.8774(3) | 0.1219(3) | 0.086(1) |
| S(2) | 0.4555(3) | 0.6370(4) | 0.4214(5) | 0.141(2) |
| O(1) | 0.2185(4) | 0.7372(6) | 0.4365(8) | 0.079(3) |
| O(2) | 0.3162(4) | 0.6363(8) | 0.5349(9) | 0.106(3) |
| O(3) | 0.3188(4) | 0.9012(5) | 0.4097(6) | 0.058(2) |
| O(4) | 0.0416(4) | 0.9429(6) | 0.3390(8) | 0.085(3) |
| O(5) | 0.8185(5) | 0.0004(8) | 0.2629(9) | 0.106(3) |
| N(1) | 0.0111(4) | 0.7607(6) | 0.4752(7) | 0.052(2) |
| C(1) | 0.2895(5) | 0.7107(9) | 0.4632(9) | 0.048(3) |
| C(2) | 0.3330(5) | 0.7876(8) | 0.3826(8) | 0.048(3) |
| C(3) | 0.3004(5) | 0.7672(8) | 0.2296(8) | 0.046(3) |
| C(4) | 0.4173(5) | 0.7650(8) | 0.4148(8) | 0.052(3) |
| C(5) | 0.1635(5) | 0.6746(9) | 0.497(1) | 0.062(3) |
| C(6) | 0.1435(5) | 0.7488(9) | 0.6085(9) | 0.057(3) |
| C(7) | 0.0989(6) | 0.6415(8) | 0.378(1) | 0.059(3) |
| C(8) | 0.0382(5) | 0.7325(9) | 0.3439(9) | 0.056(3) |
| C(9) | 0.0761(6) | 0.840(1) | 0.315(1) | 0.064(3) |
| C(10) | 0.1014(6) | 0.8974(8) | 0.443(1) | 0.060(3) |
| C(11) | 0.0785(5) | 0.8286(8) | 0.5540(9) | 0.053(3) |
| C(12) | -0.0632(6) | 0.826(1) | 0.444(1) | 0.086(4) |
| C(13) | -0.0063(6) | 0.6595(9) | 0.554(1) | 0.062(3) |
| C(14) | 0.4747(4) | 0.8652(9) | 0.430(1) | 0.030(2) |
| C(15) | 0.2839(5) | 0.6644(9) | 0.1629(9) | 0.055(3) |
| C(16) | 0.528(2) | 0.818(2) | 0.445(2) | 0.22(1) |
| C(17) | 0.5445(5) | 0.702(2) | 0.441(1) | 0.144(6) |
| C(18) | 0.2552(6) | 0.684(1) | 0.019(1) | 0.079(4) |
| C(19) | 0.2507(6) | 0.792(1) | -0.016(1) | 0.080(4) |
| H(1) | -0.0767 | 0.8453 | 0.5286 | 0.102 |
| H(2) | -0.0572 | 0.8919 | 0.3949 | 0.102 |
| H(3) | -0.1021 | 0.7810 | 0.3906 | 0.102 |
| H(4) | -0.0210 | 0.6826 | 0.6359 | 0.073 |
| H(5) | -0.0463 | 0.6178 | 0.4982 | 0.073 |
| H(6) | 0.0377 | 0.6134 | 0.5781 | 0.073 |
| H(7) | 0.1300 | 0.7026 | 0.6770 | 0.069 |
| H(8) | 0.1873 | 0.7915 | 0.6490 | 0.069 |
| H(9) | 0.1190 | 0.6284 | 0.2985 | 0.069 |
| H(10) | 0.0762 | 0.5750 | 0.4016 | 0.069 |

(fortgesetzt)

| Atom | x | y | z | u (eq) |
|---|---|---|---|---|
| H(11) | 0.1873 | 0.6082 | 0.5393 | 0.073 |
| H(12) | -0.0025 | 0.7116 | 0.2699 | 0.066 |
| H(13) | 0.1084 | 0.8383 | 0.2506 | 0.075 |
| H(14) | 0.1498 | 0.9329 | 0.4626 | 0.071 |
| H(15) | 0.0658 | 0.8734 | 0.6250 | 0.063 |
| H(16) | 0.2906 | 0.5927 | 0.2065 | 0.065 |
| H(17) | 0.2406 | 0.6258 | -0.0469 | 0.094 |
| H(18) | 0.2328 | 0.8191 | -0.1075 | 0.097 |
| H(19) | 0.4649 | 0.9443 | 0.4254 | 0.037 |
| H(20) | 0.5729 | 0.8656 | 0.4660 | 0.268 |
| H(21) | 0.5930 | 0.6651 | 0.4477 | 0.165 |
| H(22) | 0.8192 | -0.0610 | 0.1619 | 0.084 |
| H(23) | 0.7603 | 0.0105 | 0.2412 | 0.084 |

x, y, z: fraktionelle Koordinaten;
U(eq) mittlere quadratische Amplitude atomarer Bewegung im Kristall;

**C) Darstellung des mittels des erfindungsgemäßen Verfahrens erhaltenen Tiotropiumbromid-mikronisats**

Das gemäß vorstehend beschriebener Vorgehensweise erhältliche Tiotropiumbromid-monohydrat wird mit einer Luftstrahlmühle vom Typ 2-Zoll Microniser mit Mahlring 0,8 mm-Bohrung, Firma Sturtevant Inc., 348 Circuit Street, Hanover, MA 02239, USA mikronisiert. Unter Verwendung von Stickstoff als Mahlgas werden dabei beispielsweise die folgenden Mahlparameter eingestellt:

Mahldruck: 5,5 bar; Speisedruck: 5,5 bar;
Zufuhr (des kristallinen Monohydrats) bzw. Fließgeschwindigkeit: 19 g/min.

Das erhaltene Mahlgut wird anschließend auf Hordenblechen in einer Schichtdicke von etwa 1 cm ausgebreitet und für 24 - 24,5 Stunden den folgenden Klimabedingungen unterworfen: Temperatur: 25 - 30 °C; Relative Feuchte: 70-80%.

D) Meßtechniken zur Charakterisierung des mittels des erfindungsgemäßen Verfahrens erhaltenen Tiotropiumbromid-mikronisats

Die in der Beschreibung genannten, das mittels des erfindungsgemäßen Verfahrens erhaltene Tiotropiumbromid-mikronisat charakterisierendenParameter wurden gemäß den nachfolgend beschriebenen Meßtechniken und Methoden erhalten:

**D.1) Bestimmung des Wassergehalts nach Karl-Fischer (Tiotropium Bromid):**

| | |
|---|---|
| Titrator | Typ Mettler DL 18 mit |
| Kalibriersubstanz: | Dinatriumtartratdihydrat |
| Titrant: | Hydranal-Titrant 5 (Riedel-deHaen) |
| Lösungsmittel: | Hydranal Solvent (Riedel-deHaen) |

Meßmethode:

| | |
|---|---|
| Probenmenge: | 50-100 mg |
| Rührzeit: | 60 s |

Die Rührzeit vor Beginn der Titration dient dazu, die vollständige Auflösung der Probe zu gewährleisten. Der Wassergehalt der Probe wird vom Gerät in Prozent berechnet und ausgegeben.

**D.2) Partikelgrößenbestimmung mittels Laserbeugung (Fraunhoferbeugung)** Meßmethode:

Zur Bestimmung der Partikelgröße wird das Pulver mittels Dispergiereinheit einem Laserbeugungs-Spektrometer zugeführt.

| | |
|---|---|
| Meßgerät: | Laser-Beugungs-Spektrometer (HELOS),Fa. Sympatec |
| Software: | WINDOX Version 3.3/REL 1 |
| Dispergiereinheit: | RODOS / Dispergierdruck: 3 bar |

Geräteparameter:

| | |
|---|---|
| Detektor: | Multielementdetektor (31 halbkreisförmige Ringe) |
| Methode: | Luft-Dispergierung |
| Brennweite: | 100 mm |
| Messbereich: | RS 0,5/0,9 - 175 μm |
| Auswertemodus: | HRLD-Mode |

Rodos Trockendispergierer:

| | |
|---|---|
| Injektor: | 4 mm |
| Druck: | 3 bar |
| Injektor-Unterdruck: | maximal (~ 100 mbar) |
| Absaugung: | Nilfilsk (Vorlauf 5 s) |
| Dosierer: | Vibri |
| Förderrate: | 40 % (manuelle Steigerung bis 100 %) |
| Betthöhe: | 2 mm |
| Drehzahl: | 0 |

**D.3) Bestimmung der Spezifischen Oberfläche (1-Pkt.-B.E.T.-Methode):** Meßmethode:

Die Bestimmung der spezifischen Oberfläche erfolgt, indem die Pulverprobe einer Stickstoff/Heliumatmosphäre bei unterschiedlichen Drücken ausgesetzt wird. Durch Abkühlung der Probe erfolgt eine Kondensation der Stickstoffmolekülen auf der Oberfläche der Partikel. Die kondensierte Stickstoffmenge wird über die Änderung der thermischen Leitfähigkeit des Stickstoff/Heliumgemisches bestimmt und die Oberfläche der Probe über den Flächenbedarf von Stickstoff bestimmt. Über diesen Wert und die Probeneinwaage wird die spezifische Oberfläche berechnet.

Geräte und Materialien:

| | |
|---|---|
| Messgerät: | Monosorb, Fa. Quantachrome |
| Ausheizgerät: | Monotektor, Fa. Quantachrome |
| Mess- und Trockengas: | Stickstoff (5.0) / Helium (4.6) 70/30, Fa. Messer Griesheim |
| Adsorbat: | Stickstoff 30%ig in Helium |
| Kältemittel: | flüssiger Stickstoff |
| Messzelle: | mit Kapillarrohr, Fa. W. Pabisch GmbH&Co.KG |
| Kalibrierspritze; | 1000 μl, Fa. Precision Sampling Corp. |

(fortgesetzt)

| | |
|---|---|
| Analysenwaage: | R 160 P, Fa. Satorius |

Berechnung der spezifischen Oberfläche:

Die Messwerte werden vom Gerät in [$m^2$] angezeigt und werden i.d.R. in [$cm^2/g$] auf die Einwaage (Trockenmasse) umgerechnet:

$$A_{spez} = \frac{MW * 10000}{m_{tr}}$$

$A_{spez}$ = spezifische Oberfläche [$cm^2/g$]
MW = Messwert [$m^2$]
$m_{tr}$ = Trockenmasse [g]
10000 = Umrechnungsfaktor [$cm^2/m^2$]

**D.4) Bestimmung der Lösungswärme (Lösungsenthalpie) $E_c$:**

Die Bestimmung der Lösungsenthalpie erfolgt mittels eines Lösungskalorimeter *2225 Precision Solution Calorimeter* der Fa. Thermometric.
Die Lösungswärme wird anhand der - infolge des Löseprozesses - auftretenden Temperaturänderung und der aus der Basislinie berechneten systembedingten Temperaturänderung berechnet.
Vor und nach dem Ampullenbruch wird jeweils eine elektrische Kalibrierung mit einem integrierten Heizwiderstand genau bekannter Leistung durchgeführt. Hierbei wird eine bekannte Wärmeleistung über einen festgelegten Zeitraum an das System abgegeben und der Temperatursprung ermittelt.

Methoden- und Geräteparameter:

| | |
|---|---|
| Lösungskalorimeter: | 2225 Precision Solution Calorimeter, Fa. Thermometric |
| Reaktionszelle: | 100 ml |
| Thermistorwiderstand: | 30,0 kΩ (bei 25 °C) |
| Rührergeschwindigkeit: | 600 U/min |
| Thermostat: | Thermostat des 2277 Thermal Activity Monitor TAM, Fa. Thermometric |
| Temperatur: | 25 °C ± 0.0001 °C (über 24h) |
| Meßampullen: | Crushing ampoules 1 ml, Fa. Thermometric |
| Dichtung: | Silikonstopfen und Bienenwachs, Fa. Thermometric |
| Einwaage: | 40 bis 50 mg |
| Lösemittel: | Wasser, chemisch rein |
| Volumen Lösemittel: | 100 ml |
| Badtemperatur: | 25°C |
| Temperaturauflösung: | High |
| Starttemperatur: | -40mK (± 10mK) temperature-offset |
| Interface: | 2280-002 TAM accessory interface 50 Hz, Fa. Thermometric |
| Software: | SolCal V 1.1 für WINDOWS |
| Auswertung: | Automatische Auswertung mit Menüpunkt CALCULATION/ ANALYSE EXPERIMENT. (Dynamik der Basislinie ; Kalibrierung nach dem Ampullenbruch). |

Elektrische Kalibrierung:

Die elektrische Kalibrierung erfolgt während der Messung, einmal vor und einmal nach dem Ampullenbruch. Zur Auswertung wird die Kalibrierung nach dem Ampullenbruch herangezogen.

| | |
|---|---|
| Wärmemenge: | 2,5Ws |
| Heizleistung: | 250 mW |
| Heizdauer: | 10s |
| Dauer der Basislinien: | 5 min (vor und nach Heizen) |

Auswertung für Tiotropiumbromid-mikronisat:

Da die Masse des eingewogenen Tiotropiumbromid-mikronisats um den Wassergehalt des Materials korrigiert werden muss, werden die unverschlossenen Ampullen zusammen mit ca. 1 g der Testsubstanz mindestens 4 h offen stehen gelassen. Nach dieser Äquilibrierzeit werden die Ampullen mit den Silikonstopfen verschlossen und der Wassergehalt der Bulkprobe mittels Karl-Fischer-Titration bestimmt.
Die gefüllte und verschlossene Ampulle wird auf der Waage zurückgewogen. Die Korrektur der Probenmasse erfolgt nach folgender Formel:

$$m_c = \left(\frac{100\% - x}{100\%}\right) \cdot m_w$$

dabei sind:

$m_c$ korrigierte Masse
$m_w$ in die Ampulle eingewogene Probenmasse
x Wassergehalt in Prozent (mittels Karl-Fischer-Titration parallel bestimmt)

Die nach dieser Berechnung bestimmte korrigierte Masse $m_c$ wird als Eingabewert (= Einwaage) zur Berechnung der gemessenen Lösungsenthalphie verwendet.

**E) Darstellung der Pulverformulierung enthaltend das mittels des erfindungsgemäßen Verfahrens erhaltene Tiotropiumbromid-mikronisat**

In den nachfolgenden Beispielen wird als gröberer Hilfsstoff Lactose-Monohydrat (200M) verwendet. Dieser kann beispielsweise von der Firma DMV International, 5460 Veghel/NL unter der Produktbezeichnung Pharmatose 200M bezogen werden.
In den nachfolgenden Beispielen wird als feinerer Hilfsstoff Lactose-Monohydrat (5$\mu$) verwendet. Dieser kann durch gängige Verfahren (Mikronisieren) aus Lactose-Monohydrat 200M erhalten werden. Lactose-Monohydrat 200M kann beispielsweise von der Firma DMV International, 5460 Veghel/NL unter der Produktbezeichnung Pharmatose 200M bezogen werden.

Apparatives

Zur Herstellung der Tiotropiumbromid-mikronisat enthaltenden Inhalationspulver können beispielsweise die folgenden Maschinen und Geräte Verwendung finden:

Mischbehälter bzw. Pulvermischer:

Rhönradmischer 200 L; Typ: DFW80N-4; Hersteller: Firma Engelsmann, D-67059 Ludwigshafen.

Siebgranulator:

Quadro Comil; Typ: 197-S; Hersteller: Firma Joisten & Kettenbaum, D-51429 Bergisch-Gladbach.

**E.1) Herstellung der Hilfsstoffmischung:**

Als gröbere Hilfsstoffkomponente werden 31,82 kg Lactose Monohydrat für Inhalationszwecke (200M) eingesetzt. Als feinere Hilfsstoffkomponente werden 1,68 kg Lactose Monohydrat (5$\mu$m) eingesetzt. In den daraus erhaltenen 33,5 kg Hilfsstoffmischung beträgt der Anteil der feineren Hilfsstoffkomponente 5%.

Über einen geeigneten Siebgranulator mit einem Sieb mit einer Maschenweite von 0,5 mm werden in einen geeigneten Mischbehälter ca. 0,8 bis 1,2 kg Lactose Monohydrat für Inhalationszwecke (200M) vorgelegt. Anschließend werden abwechselnd Lactose Monohydrat (5$\mu$m) in Portionen von ca. 0,05 bis 0,07 kg und Lactose Monohydrat für Inhalationszwecke (200M) in Portionen von 0,8 bis 1,2 kg schichtweise eingesiebt. Lactose Monohydrat für Inhalationszwecke (200M) und Lactose Monohydrat (5$\mu$m) werden in 31 bzw. in 30 Schichten (Toleranz: ±6 Schichten) zugegeben.
Die eingesiebten Bestandteile werden anschließend gemischt (Mischen: 900 Umdrehungen)

**E.2) Herstellung der Endmischung :**

Zur Herstellung der Endmischung werden 32,87 kg der Hilfsstoffmischung (1.1) und etwa 0,13 kg des mittels des erfindungsgemäßen Verfahrens erhaltenen Tiotropiumbromid-mikronisats eingesetzt. In den daraus erhaltenen 33,0 kg Inhalationspulver beträgt der Wirkstoffanteil 0.4%.
Über einen geeigneten Siebgranulator mit einem Sieb mit einer Maschenweite von 0,5 mm werden in einen geeigneten Mischbehälter ca. 1,1 bis 1,7 kg Hilfsstoffmischung (E.1) vorgelegt. Anschließend werden abwechselnd Tiotropiumbromid-mikronisat in Portionen von ca. 0,003 kg und Hilfsstoffmischung (E.1) in Portionen von 0,6 bis 0,8 kg schichtweise eingesiebt. Die Zugabe der Hilfststoffmischung und des Wirkstoffs erfolgt in 46 bzw. 45 Schichten (Toleranz: ± 9 Schichten).
Die eingesiebten Bestandteile werden anschließend gemischt (Mischen: 900 Umdrehungen). Die Endmischung wird noch zweimal über einen Siebgranulator gegeben und anschließend jeweils gemischt (Mischen: 900 Umdrehungen).

**E.3) Inhalationskapseln:**

Mit der nach E.2 erhaltenen Mischung werden Inhalationskapseln (Inhaletten) der folgenden Zusammensetzung erhalten:

| | |
|---|---|
| Tiotropiumbromid-mikronisat: | 0,0225 mg |
| Lactose Monohydrat (200 M): | 5,2025 mg |
| Lactose Monohydrat (5$\mu$m): | 0,2750 mg |
| Hartgelatinekapsel : | 49,0 mg |
| Total: | 54,5 mg |

Unter analoger Anwendung der in E.2 beschriebenen Vorgehensweise werden ferner Inhalationskapseln (Inhaletten) der folgenden Zusammensetzung erhalten:
a)

| | |
|---|---|
| Tiotropiumbromid-mikronisat: | 0,0225 mg |
| Lactose Monohydrat (200 M): | 4,9275 mg |
| Lactose Monohydrat (5 $\mu$m): | 0,5500 mg |
| Hartgelatinekapsel: | 49,0 mg |
| Total: | 54,5 mg |

b)

| | |
|---|---|
| Tiotropiumbromid-mirkonisat: | 0,0225 mg |
| Lactose Monohydrat (200 M): | 5,2025 mg |
| Lactose Monohydrat (5 $\mu$m) : | 0,2750 mg |
| Polyethylenkapsel: | 100,0 mg |
| Total: | 105,50 mg |

**F) Meßtechniken zur Partikelgrößenbestimmung der Hilfsstoffkomponenten, die In E) zur Anwendung gelangen**

Nachfolgend wird beschrieben, wie die Bestimmung der mittleren Teilchengröße der verschiedenen Hilfsstoffbestandteile der das erfindungsgemäße Tiotropiumbromid-mikronisat enthaltenden und nach E) darstellbaren Formulierung erfolgen kann.

**F.1) Partikelgrößenbestimmung von feinteiliger Lactose:** Meßgerät und Einstellungen:

Die Bedienung der Geräte erfolgt in Übereinstimmung mit den Bedienungsanleitungen des Herstellers.

| | |
|---|---|
| Meßgerät: | HELOS Laser-Beugungs-Spektrometer, (SympaTec) |
| Dispergiereinheit: | RODOS Trockendispergierer mit Saugtrichter, (SympaTec) |
| Probenmenge: | ab 100 mg |
| Produktzufuhr: | Schwingrinne Vibri, Fa. Sympatec |
| Frequenz d. Vibrationsrinne: | 40 bis 100 % ansteigend |
| Dauer der Probenzufuhr: | 1 bis 15 sek. (im Fall von 100 mg) |
| Brennweite: | 100 mm (Meßbereich: 0,9 - 175 $\mu$m) |
| Meßzeit: | ca. 15 s (im Fall von 100 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Mind. 100 mg der Prüfsubstanz werden auf einem Kartenblatt eingewogen. Mit einem weiteren Kartenblatt werden alle größeren Agglomerate zerstoßen. Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne von ca. 40 % bis 100 % (gegen Ende der Messung) variiert. Die Zeit, in der jeweils die gesamte Probe zugeführt wird beträgt 10 bis 15 sek.

**F.2) Partikelgrößenbestimmung von Laktose 200M:**

Meßgerät und Einstellungen

Die Bedienung der Geräte erfolgte in Übereinstimmung mit den Bedienungsanleitungen des Herstellers.

| | |
|---|---|
| Meßgerät: | Laser-Beugungs-Spektrometer (HELOS), Sympatec |
| Dispergiereinheit: | Trockendispergierer RODOS mit Saugtrichter, Sympatec |
| Probenmenge: | 500 mg |
| Produktzufuhr: | Vibrationsrinne Typ VIBRI , Sympatec |
| Frequenz d. Vibrationsrinne: | 18 bis 100 % ansteigend |
| Brennweite (1): | 200 mm (Meßbereich: 1.8 - 350 $\mu$m) |
| Brennweite (2): | 500 mm (Meßbereich: 4.5 - 875 $\mu$m) |
| Meßzeit/Wartezeit: | 10 s |
| Zykluszeit: | 10 ms |
| Start/Stop bei: | 1 % auf Kanal 19 |
| Druck: | 3 bar |
| Unterdruck: | maximal |
| Auswertemodus: | HRLD |

Probenvorbereitung / Produktzufuhr:

Ca. 500 mg der Prüfsubstanz werden auf einem Kartenblatt eingewogen. Mit einem weiteren Kartenblatt werden alle größeren Agglomerate zerstoßen. Das Pulver wird in der Trichter der Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne von 0 auf 40 % gesteigert bis sich ein kontinuierlicher Produktfluß einstellt. Danach wird auf eine Amplitude von ca. 18% reduziert. Gegen Ende der Messung wird die Amplitude auf 100% gesteigert.

## Patentansprüche

1. Verfahren zur Herstellung des kristallinen Tiotroplumbromid-Mikronisats der Formel (I) **gekennzeichnet durch** eine Partikelgröße $X_{50}$ von zwischen 1,0 μm und 3.5 μm bei einem Wert $Q_{(5.8)}$ von größer 60%, **durch** einen spezifischen Oberflächenwert im Bereich zwischen 2 $m^2/g$ und 5 $m^2/g$, **durch** eine spezifische Lösungswärme von größer 65 Ws/g sowie **durch** einen Wassergehalt von 1 % bis 4,5 %, **dadurch** gekennzeichnet, daß

   a) kristallines Tiotrophimbromid-Monohydrat, welches bei der thermischen Analyse mittels OSC ein endothermes Maximum bei 230 ± 5°C bei einer Heizrate von 10K/min aufweist, welches **durch** ein IR-Spektrum gekennzeichnet ist, das unter anderen bei den Wellenzahlen 3570, 3410, 3105, 1730, 1260, 1035 und 720 $cm^{-1}$ Banden aufweist und welches **durch** eine einfache monoklinische Zelle mit folgenden Dimensionen: a = 18.0774 Å, b = 11.9711 Å, c = 9.9321 Å, β = 102.891°, V = 2096.96 $Å^3$ gekennzeichnet ist, mikronisiert und
   b) anschließend bei einer Temperatur von 15 - 40 °C Wasserdampf einer relativen Feuchte von wenigstens 40% für einen Zeltraum von wenigstens 6 Stunden ausgesetzt wird.

2. Verfahren nach Anspruch 1, **dadurch gekennzeichnet, daß** zur Durchführung des Schritts a) unter Inertgas, vorzugsweise Sticktoff mikronisiert wird.

3. Verfahren nach Anspruch 1 oder 2 **dadurch gekennzeichnet, daß** zur Durchführung des Schritts a) eine Luftstrahimühle unter Verwendung der folgenden Mahlparameter zum Einsatz gelangt:

| | |
|---|---|
| Mahldruck: | 2-8 bar; |
| Speisedruck: | 2-8 bar; |
| Mahlgas/Speisegas: | Stickstoff; |
| Produktzufuhr: | 5-35 g/min. |

4. Verfahren nach einem der Ansprüche 1, 2 oder 3, **dadurch gekennzeichnet, daß** zur Dufchführung des Schritts b) das aus Schritt a) erhaltene Produkt bei einer Temperatur von 20 - 35 °C Wasserdampf einer relativen Feuchte von 50-95% für einen Zeitraum von 12 bis 48 Stunden ausgesetzt wird.

5. Verfahren nach einem der Ansprach 1 bis 4, **dadurch gekennzeichnet, daß** das als Ausgangsprodukt zum Einsatz gelangende kristalline Tiotropiumbromid-Monohydrat durch die nachfolgenden Schritte erhalten wird:

   a) Aufnahme von Tiotroplumbromid in Wasser:
   b) Erwärmen der erhaltenen Mischung;
   c) Zugabe von Aktivkohle und
   d) nach Abtrennen der Aktivkohle langsames Kristallisieren des Tiotropiumbromid-monohydrats unter langsamem Abkühlen der wässrigen Lösung.

6. Verfahren gemäß Anspruch 5, **dadurch gekennzeichnet, daß**

   a) pro Mol eingesetztes Tiotropiumbromid 0,4 bis 1,5 kg Wasser verwendet werden,
   b) die erhaltene Mischung auf mehr als 50°C erwärmt wird,
   c) pro Mol eingesetztes Tiotropiumbromid 10 bis 50 g Aktivkohle eingesetzt werden und nach erfolgter Aktivkohlezugabe zwischen 5 bis 60 Minuten, weitergerührt wird,
   d) die erhaltene Mischung filtriert, das erhaltene Filtrat mit einer Abkühlrate von 1 bis 10°C pro 10 bis 30 Minuten auf eine Temperatur von 20-25°C abgekühlt und das Tiotropiumbromid-monohydrat dabei kristallisiert wird.

## Claims

1. Process for preparing the crystalline tiotropium bromide micronisate of formula (I),

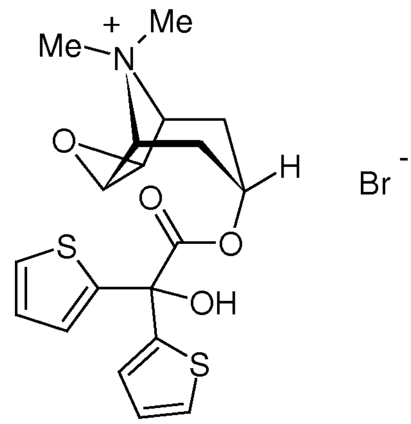

(I)

**characterised by** a particle size $X_{50}$ of between 1.0 μm and 3.5 μm at a $Q_{(5.8)}$ value of more than 60%, by a specific surface value in the range between 2 $m^2/g$ and 5 $m^2/g$, by a specific heat of solution of more than 65 Ws/g and by a water content of 1 % to 4.5 %, **characterised in that**

a) crystalline tiotropium bromide monohydrate, which when thermally analysed by DSC has an endothermic maximum at 230 ± 5°C at a heating rate of 10K/min, which is **characterised by** an IR spectrum which has bands *inter alia* at wavelengths 3570, 3410, 3105, 1730, 1260, 1035 and 720 $cm^{-1}$ and which is **characterised by** a simple monoclinic cell with the following dimensions: a = 18.0774 Å, b = 11.9711 Å, c = 9.9321 Å, β = 102.691°, V = 2096.96 $Å^3$, is micronised and

b) then at a temperature of 15 - 40 °C exposed to water vapour with a relative humidity of at least 40% for a period of at least 6 hours.

2. Process according to claim 1, **characterised in that** in order to carry out step a) the micronisation is carried out under inert gas, preferably nitrogen.

3. Process according to claim 1 or 2, **characterised in that** to carry out step a) an air jet mill is used with the following grinding parameters:

| | |
|---|---|
| grinding pressure: | 2 - 8 bar; |
| feed pressure: | 2 - 8 bar; |
| grinding gas/feed gas: | nitrogen; |
| product supply: | 5 - 35 g/min. |

4. Process according to one of claims 1, 2 or 3, **characterised in that** in order to carry out step b) the product obtained from step a) at a temperature of 20 - 35°C is exposed to water vapour at a relative humidity of 50 - 95% for a period of 12 to 48 hours.

5. Process according to one of claims 1 to 4, **characterised in that** the crystalline tiotropium bromide monohydrate used as starting product is obtained by the following steps:

a) taking up tiotropium bromide in water;
b) heating the resulting mixture;
c) adding activated charcoal and
d) after removing the activated charcoal, slowly crystallising the tiotropium bromide monohydrate while slowly cooling the aqueous solution.

6. Process according to claim 5, **characterised in that**

a) 0.4 to 1.5 kg of water are used per mol of tiotropium bromide used,
b) the mixture obtained is heated to more than 50°C,
c) 10 to 50 g of activated charcoal are used per mol of tiotropium bromide used and after the activated charcoal has been added the mixture is stirred for between 5 and 60 minutes,
d) the mixture obtained is filtered, the filtrate obtained is cooled to a temperature of 20-25°C at a cooling rate of from 1 to 10°C per 10 to 30 minutes and the tiotropium bromide monohydrate is crystallised.

## Revendications

1. Procédé pour la production du micronisat cristallin de bromure de tiotropium de formule (I)

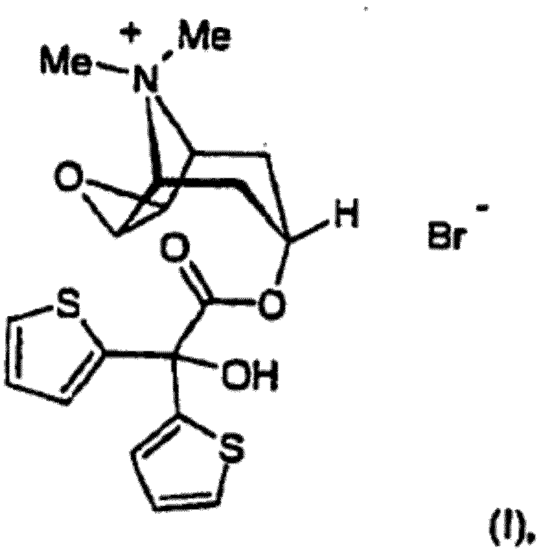

(I),

**caractérisé par** une taille de particule $X_{50}$ entre 1,0 μm et 3,5 μm, pour une valeur $Q_{(5.8)}$ supérieure à 60 %, par une valeur de surface spécifique dans la plage entre 2 $m^2/g$ et 5 $m^2/g$, par une chaleur de solution spécifique supérieure à 65 Ws/g ainsi que par une teneur en eau de 1 % à 4,5 %, **caractérisé en ce que**

a) le monohydrate cristallin de bromure de tiotropium qui présente un maximum endothermique de 230 ± 5 °C à une vitesse de chauffage de 10 K/min lors de l'analyse thermique par DSC, qui est **caractérisé par** un spectre IR qui présente entre autre des bandes aux nombres d'ondes 3570, 3410, 3105, 1730, 1260, 1035 et 720 $cm^{-1}$ et qui est **caractérisé par** une maille monoclinique simple ayant les dimensions suivantes : a = 18,0774 Å, b = 11,9711 Å, c = 9,9321 Å, β = 102,691°, V = 2096,96 Å$^3$, est micronisé et
b) ensuite soumis, à une température de 15 à 40 °C, à de la vapeur d'eau ayant une humidité relative d'au moins 40 % pendant une durée d'au moins 6 heures.

2. Procédé selon la revendication 1, **caractérisé en ce que**, pour la mise en oeuvre de l'étape a), on micronise sous gaz inerte, de préférence l'azote.

3. Procédé selon la revendication 1 ou 2, **caractérisé en ce que**, pour la mise en oeuvre de l'étape a), on utilise un broyeur à jet d'air en utilisant les paramètres de broyage suivants :

pression de broyage : 2 à 8 bars ;
pression d'alimentation : 2 à 8 bars ;
gaz de broyage/gaz d'alimentation : azote ;
amenée de produit : 5 à 35 g/min.

4. Procédé selon l'une des revendications 1, 2 ou 3, **caractérisé en ce que**, pour la mise en oeuvre de l'étape b), le produit obtenu à l'étape a) est exposé à une température de 20 à 35 °C à de la vapeur d'eau ayant une humidité relative de 50 à 95 % pendant une durée de 12 à 48 heures.

5. Procédé selon l'une des revendications 1 à 4, **caractérisé en ce que** le monohydrate cristallin de bromure de tiotropium employé comme produit de départ est obtenu par les étapes suivantes :

a) introduction de bromure de tiotropium dans l'eau ;
b) chauffage du mélange obtenu ;
c) addition de charbon actif et
d) après séparation du charbon actif, cristallisation lente du monohydrate de bromure de tiotropium en refroidissant lentement la solution aqueuse.

6. Procédé selon la revendication 5, **caractérisé par**

a) par mole de bromure de tiotropium employé, on utilise de 0,4 à 1,5 kg d'eau,
b) le mélange obtenu est chauffé à plus de 50 °C,
c) par mole de bromure de tiotropium employé, on utilise 10 à 50 g de charbon actif et, après l'addition effective du charbon actif, on continue à agiter entre 5 à 60 minutes,
d) on filtre le mélange obtenu, on refroidit le filtrat obtenu à une température de 20 à 25 °C à une vitesse de refroidissement de 1 à 10 °C pour 10 à 30 minutes et, on cristallise ainsi le monohydrate de bromure de tiotropium.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 418716 A1
- FR 2779347
- US 4570630 A
- DE 3625685 A
- WO 9428958 A